# EUROPEAN PATENT APPLICATION

(11) **EP 2 742 855 A1**
(43) Date of publication of application: **18.06.2014**
(21) Application number: 12196603.0
(22) Date of filing: 11.12.2012
(51) Int. Cl.: A61B 5/00, A61B 5/055, A61B 6/03

(54) **Brain functional magnetic resonance activity is associated with response to tumor necrosis factor inhibition**

(71) Applicant: Friedrich-Alexander-Universität Erlangen-Nürnberg, 91054 Erlangen (DE); UNIVERSITÄTSKLINIKUM ERLANGEN, 91054 Erlangen (DE)
(72) Inventor: Schett, Georg, 91054 Erlangen (DE); Hess, Andreas, 91080 Uttenreuth (DE)
(74) Representative: Vossius & Partner

(57) **Abstract**

The present invention relates to a non-invasive method for predicting the responsiveness of a human subject suffering from inflammatory disease to a treatment with a therapy against said inflammatory disease using brain imaging techniques. Furthermore, the present invention relates to a pharmaceutical composition comprising an active ingredient for the treatment of human subjects suffering from inflammatory disease and being identified as responders to therapy against the inflammatory disease according to the method of the invention. Preferably, this invention relates to a non-invasive method for predicting the responsiveness of a human subject suffering from rheumatoid arthritis to a treatment with TNF-antagonists comprising at least one step of brain imaging, preferably with an fMRI apparatus. The present invention shows that response to TNFi depends on the gestalt of brain activity in rheumatoid arthritis (RA) patients.

## Description

The present invention relates to a non-invasive method for predicting the responsiveness of a human subject suffering from inflammatory disease to a treatment with a therapy against said inflammatory disease using brain imaging techniques. Furthermore, the present invention relates to a pharmaceutical composition comprising an active ingredient for the treatment of human subjects suffering from inflammatory disease and being identified as responders to therapy against the inflammatory disease according to the method of the invention. Preferably, this invention relates to a non-invasive method for predicting the responsiveness of a human subject suffering from rheumatoid arthritis to a treatment with TNF-antagonists comprising at least one step of brain imaging, preferably with an FMRI apparatus. The present invention shows that response to TNFi depends on the gestalt of brain activity in rheumatoid arthritis (RA) patients.

A number of documents including patent applications, manufacturer's manuals and scientific publications are cited herein. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Rheumatoid arthritis (RA) is an autoimmune inflammatory disease affecting approximately 1% of the population worldwide. The disease which affects particularly women, is characterized by loss of immune tolerance against self-proteins followed by persistent inflammation of multiple joints. RA rapidly leads to the destruction of the cartilage and bone of the affected joints which is associated with functional decline and premature death (see, e.g., Mclnnes (2011) N Engl J Med. 365:2205-2219 or Firestein (2003) Nature 423:356-361). Treatment of RA has been extremely challenging before the introduction of agents neutralizing the biological activity of tumor necrosis factor (TNF), because standard anti-inflammatory drug therapy shows either limited efficacy in reducing inflammation or has proven as rather toxic in its long-term use.

Development of TNF inhibitors (TNFi) has substantially changed this unmet therapeutic need and dramatically improved treatment of several different inflammatory diseases (see Feldmann (1996) Cell 85:307-310). In this context it is particularly stunning that neutralization of a single pro-inflammatory cytokine, namely TNF, is highly effective in treating rather complex disease processes such as RA, psoriasis and inflammatory bowel disease. Even more interesting, improvement of signs and symptoms of disease occurs rapidly after the start of TNFi, even before remission of inflammation becomes clinically measureable. In this context, we have recently shown that both mice with TNF-mediated arthritis and humans with RA show enhanced brain activity in centers involved in pain perception and processing and the control of emotions (see Hess (2011) Proc Natl Acad Sci U S A. 108:3731-3736). TNFi treatment reverses this enhanced central nervous system activity in both mice and humans as early as 24 hours, again even before clinical effects on arthritis are observed. These data suggest that TNFi can rapidly turn down pathologically increased neuronal activity in the brain, which is elicited by peripheral inflammatory disease. These central nervous effects of TNFi are further substantiated by positron-emission tomography studies suggesting that TNFi change serotonin transporter expression in the brain within the first weeks of therapy (see Cavanagh (2010) Ann Rheum Dis. 69:1251-1252). Since key outcome measures in RA are based on the patients' disease perception, rather than objective measures (see Prevoo (1995) Arthritis Rheum 1995; 38: 44-48), fast improvement of disease symptoms in RA patients receiving TNFi is not surprising, and led to the hypothesis that interference with reflex arches between the peripheral inflammatory process and the central nervous system (see Tracey (2007) J Clin Invest. 117:289-229) is crucial for explaining the rapid effects of cytokine blockade by TNFi (see Diamond (2011) Proc Natl Acad Sci USA 108: 3461-3462). Considering that only about 50% of RA patients experience major improvement of disease by TNFi, it has been always a key goal to better predict treatment responses (see SMolen (2003) Nat Rev Drug Discov. 2: 473-488).

Despite numerous attempts, clinical disease parameters as well as genetic and laboratory measures failed to identify characteristics allowing predicting response to TNFi (see Conaghan (2011) Clin Rheumatol. 30: S41-47 and Smolen (2008) Arthritis Res Ther.10:208). In consequence, TNFi therapy is still initiated on the basis of a "Try-and-Error Concept" in the individual patient, which is more than challenging considering the side effects of TNFi, such as increased infectious risk, as well as the high costs of the therapy. Each of the three major TNFi, approved for the treatment of RA, creates annual revenues of more than 5 billion dollars and two additional TNFi have just been approved for the treatment of RA (see Maggon, 2011, Knol Publishing, Top Ten/Twenty Best Selling Drugs 2009: World Best Selling Human Medicinal Brands 2009, Top Ten, Top Twenty, First Global Market Report, Version 69). Exact prediction of treatment response to TNFi is therefore not only of importance for the patients being exposed to these drugs but also relevant for limiting the substantial socioeconomic burden of this drug therapy.

Accordingly, there is a need for a prediction of the responsiveness of patients to therapy of inflammatory diseases. Thus, the technical problem underlying the present invention is to provide means and methods to predict the responsiveness of patients suffering from an inflammatory disease to a treatment against said inflammatory disease.

The solution to this technical problem is provided by the embodiments as defined herein and as characterized in the claims.

In accordance with this invention, a non-invasive method for predicting a response to a therapy against inflammatory disease for a human subject suffering from said inflammatory disease prior to application of said therapy against inflammatory disease using digital imaging techniques comprising the steps of:
a) collecting first digital imaging data indicative of neural activity from said human subject suffering from inflammatory disease under standardized control conditions and automatically determining, from said first digital imaging data, a value X of an activated total brain area of said human subject suffering from inflammatory disease:
b) collecting second digital imaging data indicative of neural activity from said human subject suffering from inflammatory disease upon mechanical compression of a body part affected by said inflammatory disease and automatically determining, from said second digital imaging data, a value Y of an activated total brain area of said human subject suffering from inflammatory disease;
c) calculating, from said determined values X and Y, a fractional change (X-Y)/Y;
d) automatically outputting, at an output unit, a signal representing said fractional change (X-Y)/Y;
   wherein if said fractional change (X-Y)/Y is lower than 3 and/or said fractional change (X-Y)/Y is lower than the corresponding mean fractional change as determined according to steps a) to d) in a cohort of control patients suffering from said inflammatory disease who are not responding to said therapy against inflammatory disease, then the signal output is such that it is predictive of a favourable response to therapy against inflammatory disease in the human subject suffering from said inflammatory disease was found.

Accordingly, based on the observations on the tight interaction between central nervous system activity and the peripheral inflammatory disease process the present invention relates to a radically different concept in predicting clinical response to TNFi than practiced in the prior art. The present invention uses the very surprising technical feature that a response to treatment depends on the intensity of representation of pain in the central nervous system rather than the objective clinical pattern of disease. To illustrate the power of the present invention, it has been shown by functional magnetic resonance imaging (FMRI) performed on the day before TNFi exposure in patients suffering from active RA that the invention allows separating therapy responders from non-responders (see Examples of the present invention). fMRI non-invasively detects tiny changes in hemoglobin oxygenation in the brain by measuring the blood oxygen level dependent (BOLD) signal. This signal changes due to oxygen consumption after increased neuronal activity (Ogawa (1990) Proc Natl Acad USA 87: 9868-9872).

The above identifies the technical problem of providing means and methods to predict the responsiveness of patients suffering from an inflammatory disease to a treatment against said inflammatory disease is solved by the embodiments as characterized in the claims and laid down in the appended examples. In particular, the solution is of relevance for predicting the responsiveness of patients suffering from rheumatoid arthritis to a treatment with (a) TNF antagonist(s).

The term "inflammatory disease" according to the present invention may preferably relate to rheumatoid arthritis, psoriatic arthritis, spondyloarthritis or inflammatory bowel disease.

The term "treatment against inflammatory disease" according to this invention may preferably relate to the administration of an antagonist of TNFalpha, IL-6R, IL-6 or IL-17 or administration of Adalimumab, Certolizumab pegol, Etanercept, Golimumab, Infliximab, Tocilizumab, Anakinra, Rituximab, Abatacept or Secukinumab.

The term "digital imaging techniques" according to the present invention may preferably relate to FMRI (Peyron (2000) Neurophysiol Clin 30: 263-88) using blood-oxygen level dependent (BOLD; Ogawa (1990) Proc Natl Acad USA 87: 9868-9872), arterial spin labelling (ASL; Owen (2008) Pain 136:85-96), cerebral blood flow (CBF; Owen (2008) Pain 136:85-96), or cerebral blood volume (CBV; Shih (2012) Experimental Neurology 234 (2012) 382-388) and PET (Peyron (2000) Neurophysiol Clin 30: 263-88), SPECT, EEG, MEG (Jones (1999) Rheumatic Disease Clinics of North America 25(1): 123-157).

The term "total activated brain area" according to the present invention relates to the activated cerebral regions of the brain.

According to the present invention, the person skilled in the art could use instead of the "total activated brain area" also the activated area of the thalamus, primary somatosensory cortex, secondary somatosensory cortex, parietal association cortex, posterior cingulate cortex, anterior cingulate cortex, medial prefrontal cortex, lateral prefrontal cortex, anterior insula, anterior insula, cerebellum and motor cortex according to any of the methods of the present invention.

The term "total activated brain area" also comprises the total activated brain volume. The term "standardized control conditions" according to the present invention may relate to the method of finger-tapping, which is known to the person skilled in the art. Finger-tapping may be described as non painful.

The term "mechanical compression of a body part affected by the inflammatory disease" may preferably be understood as applying a mild painful stimulus by compression of said affected body part.

Steps (a) and (b) of any of the methods of the present invention may be repeated several times in order to arrive at mean values for X and Y which can then further be used as values X and Y in steps (c) and (d) of the respective method of the present invention,

The present invention also relates to a non-invasive method for predicting a response to a therapy against inflammatory disease for a human subject suffering from said inflammatory disease prior to application of said therapy against inflammatory disease using digital imaging techniques comprising the steps of:
a) collecting first digital imaging data indicative of neural activity from said human subject suffering from inflammatory disease under standardized control conditions and automatically determining, from said first digital imaging data, a value X of an activated total brain area of said human subject suffering from inflammatory disease:
b) collecting second digital imaging data indicative of neural activity from said human subject suffering from inflammatory disease upon mechanical compression of a body part affected by said inflammatory disease and automatically determining, from said second digital imaging data, a value Y of an activated total brain area of said human subject suffering from inflammatory disease;
c) calculating, from said determined values X and Y, a fractional change (X-Y)/Y;
d) automatically outputting, at an output unit, a signal representing said fractional change (X-Y)/Y;
   wherein if said fractional change (X-Y)/Y is lower than 3, then the signal output is such that it is predictive of a favourable response to therapy against inflammatory disease in the human subject suffering from said inflammatory disease.

Further, present invention also relates to a non-invasive method for predicting a response to a therapy against inflammatory disease for a human subject suffering from said inflammatory disease prior to application of said therapy against inflammatory disease using digital imaging techniques comprising the steps of:
a) collecting first digital imaging data indicative of neural activity from said human subject suffering from inflammatory disease under standardized control conditions and automatically determining, from said first digital imaging data, a value X of an activated total brain area of said human subject suffering from inflammatory disease:
b) collecting second digital imaging data indicative of neural activity from said human subject suffering from inflammatory disease upon mechanical compression of a body part affected by said inflammatory disease and automatically determining, from said second digital imaging data, a value Y of an activated total brain area of said human subject suffering from inflammatory disease;
c) calculating, from said determined values X and Y, a fractional change (X-Y)/Y;
d) automatically outputting, at an output unit, a signal representing said fractional change (X-Y)/Y;
   wherein if said fractional change (X-Y)/Y is lower than 2.5, more preferred lower than 2, even more preferred lower than 1.5 and most preferred lower than 1, then the signal output is such that it is predictive of a favourable response to therapy against inflammatory disease in the human subject suffering from said inflammatory disease.

Further, the present invention also relates to a non-invasive method for predicting a response to a therapy against inflammatory disease for a human subject suffering from said inflammatory disease prior to application of said therapy against inflammatory disease using digital imaging techniques comprising the steps of:
a) collecting first digital imaging data indicative of neural activity from said human subject suffering from inflammatory disease under standardized control conditions and automatically determining, from said first digital imaging data, a value X of an activated total brain area of said human subject suffering from inflammatory disease:
b) collecting second digital imaging data indicative of neural activity from said human subject suffering from inflammatory disease upon mechanical compression of a body part affected by said inflammatory disease and automatically determining, from said second digital imaging data, a value Y of an activated total brain area of said human subject suffering from inflammatory disease;
c) calculating, from said determined values X and Y, a fractional change (X-Y)/Y;
d) automatically outputting, at an output unit, a signal representing said fractional change (X-Y)/Y;
   wherein, if said fractional change (X-Y)/Y is lower than the corresponding mean fractional change as determined according to steps a) to d) in a cohort of control patients suffering from said inflammatory disease who are not responding to said therapy against inflammatory disease, then such is predictive of a favourable response to said therapy against inflammatory disease in said human subject suffering from said inflammatory disease.

Furthermore, the present invention also relates to a non-invasive method for predicting a response to a therapy against inflammatory disease for a human subject suffering from said inflammatory disease prior to application of said therapy against inflammatory disease using digital imaging techniques comprising the steps of:
a) collecting first digital imaging data indicative of neural activity from said human subject suffering from inflammatory disease under standardized control conditions and automatically determining, from said first digital imaging data, a value X of an activated total brain area of said human subject suffering from inflammatory disease:
b) collecting second digital imaging data indicative of neural activity from said human subject suffering from inflammatory disease upon mechanical compression of a body part affected by said inflammatory disease and automatically determining, from said second digital imaging data, a value Y of an activated total brain area of said human subject suffering from inflammatory disease;
c) calculating, from said determined values X and Y, a fractional change (X-Y)/Y;
d) automatically outputting, at an output unit, a signal representing said fractional change (X-Y)/Y;
   wherein, if said fractional change (X-Y)/Y is by 10, 12, 14, 16, 18 or 19 %, more preferred by 20 or 25 %, even more preferred by 30 or 35 %, even further more preferred by 40, 50, 60, 70, 80, 90 %, or even furthermore preferred by 100% lower than the corresponding mean fractional change as determined according to steps a) to d) in a cohort of control patients suffering from said inflammatory disease who are not responding to said therapy against inflammatory disease, then such is predictive of a favourable response to said therapy against inflammatory disease in said human subject suffering from said inflammatory disease.

The present invention allows determining the fractional change (X-Y)/Y according to steps (a) to (d) of any of the methods of the present invention in a cohort of control patients suffering from said inflammatory disease who are not responding to said therapy against inflammatory disease as illustrated by the examples of the present invention.

The methods of the present invention relate in particular to determining the responsiveness of a patient suffering from rheumatoid arthritis, psoriatic arthritis, spondyloarthritis or inflammatory bowel disease to a treatment against said disease. The present invention relates in particular to the use of FMRI as an imaging technique according to some methods of the present invention.

Further, the present invention relates in particular to the use of fMRI as an imaging technique using blood-oxygen level dependent (BOLD; Ogawa (1990) Proc Natl Acad USA 87: 9868-9872), arterial spin labelling (ASL; Owen (2008) Pain 136:85-96), cerebral blood flow (CBF; Owen (2008) Pain 136:85-96), or cerebral blood volume (CBV; Shih (2012) Experimental Neurology 234 (2012) 382-388) and PET (Peyron (2000) Neurophysiol Clin 30: 263-88) according to some methods of the present invention.

The present invention also relates to using PET (Peyron (2000) Neurophysiol Clin 30: 263-88) as an imaging technique according to some methods of the present invention.

Further, the present invention relates in particular to the use of PET as an imaging technique using ¹⁸FDG-PET (Ido (1978) J Labeled Compounds Radiopharm 24: 174- 183) or ¹⁵O-PET (Hamdy (1999) J Neurophysiol 81: 1917-1924 and Jones (2012) Curr Rheumatol Rep 14(6): 557-67) according to some methods of the present invention.

The present invention relates in particular to determining the responsiveness of patients suffering from inflammatory disease to a treatment comprising the administration of an antagonist of TNFalpha, IL-6R, IL-6 or IL-17 according to some methods of the present invention.

The present invention also relates to determining the responsiveness of patients suffering from inflammatory disease to a treatment comprising the administration of Adalimumab, Certolizumab pegol, Etanercept, Golimumab, Infliximab, Tocilizumab, Anakinra, Rituximab, Abatacept or Secukinumab according to some methods of the present invention.

The present invention also relates to predicting the responsiveness of patients suffering from rheumatoid arthritis or psoriatic arthritis to a therapy against rheumatoid arthritis or psoriatic arthritis using the compression of the metacarpophalangeal joint as a method of "compression of a body part affected by inflammatory disease".

The present invention also relates to predicting the responsiveness of patients suffering from inflammatory bowel disease to a therapy against inflammatory bowel disease using the compression of the abdomen as a method of "compression of a body part affected by inflammatory disease" according to some methods of the present invention.

Further, according to any of the methods of the present invention, finger-tapping of the human subject suffering from inflammatory disease can be used as a standardized control condition.

The present invention further relates to a pharmaceutical composition comprising an antibody targeting TNFalpha, IL-6R, IL-6 or IL-17 as active ingredient for use in treating a human subject suffering from inflammatory disease wherein for said human subject a favourable response to therapy has been predicted according to any of the methods of the present invention.

Further, the present invention also relates to a pharmaceutical composition comprising an agent selected from the group comprising Adalimumab, Certolizumab pegol, Etanercept, Golimumab, Infliximab, Tocilizumab, Anakinra, Rituximab and Abatacept or Secukinumab for use in treating a human subject suffering from inflammatory disease wherein for said human subject a favourable response to therapy has been predicted according to any of the methods of the present invention.

In particular, the present invention also relates to a pharmaceutical composition comprising an agent selected from the group comprising Adalimumab, Certolizumab pegol, Etanercept, Golimumab, Infliximab, Tocilizumab, Anakinra, Rituximab and Abatacept or Secukinumab for use in treating a human subject suffering from rheumatoid arthritis and wherein for said human subject a favourable response to therapy has been predicted according to any of the methods of the present invention.

In particular, the present invention relates to a non-invasive method for predicting a response to TNFi therapy for a human subject suffering rheumatoid arthritis prior to application of TNFi therapy using digital imaging techniques comprising the steps of:
a) collecting first digital imaging data indicative of neural activity from said human subject suffering from rheumatoid arthritis under standardized control conditions, preferably using the method of finger-tapping known to the person skilled in the art, and automatically determining, from said first digital imaging data, a value X of an activated total brain area of said human subject rheumatoid arthritis;
b) collecting second digital imaging data indicative of neural activity from said human subject suffering from rheumatoid arthritis upon mechanical compression of a body part affected by rheumatoid arthritis, preferably by compression of the metacarpophalangeal joint, and automatically determining, from said second digital imaging data, a value Y of an activated total brain area of said human subject suffering from rheumatoid arthritis;
c) calculating, from said determined values X and Y, a fractional change (X-Y)/Y;
d) automatically outputting, at an output unit, a signal representing said fractional change (X-Y)/Y;
   wherein if said fractional change (X-Y)/Y is lower than 3 and/or said fractional change (X-Y)/Y is lower than the corresponding mean fractional change as determined according to steps a) to d) in a cohort of control patients suffering from rheumatoid arthritis who are not responding to TNFi, then the signal output is such that it is predictive of a favourable response to therapy against inflammatory disease in the human subject suffering from said inflammatory disease.

The present invention also relates to a non-invasive method for predicting a response to a therapy against inflammatory disease for a human subject suffering from said inflammatory disease prior to application of said therapy against inflammatory disease using digital imaging techniques comprising the steps of:
a) collecting first digital imaging data indicative of neural activity from said human subject suffering from inflammatory disease under standardized control conditions and automatically determining, from said first digital imaging data, a value X of an activated total brain area of said human subject suffering from inflammatory disease:
b) collecting second digital imaging data indicative of neural activity from said human subject suffering from inflammatory disease after a mechanical compression of a body part affected by said inflammatory disease had been applied to said human subject suffering from inflammatory disease or was applied by the human subject suffering from inflammatory disease itself, and automatically determining, from said second digital imaging data, a value Y of an activated total brain area of said human subject suffering from inflammatory disease;
c) calculating, from said determined values X and Y, a fractional change (X-Y)/Y;
d) automatically outputting, at an output unit, a signal representing said fractional change (X-Y)/Y;
   wherein if said fractional change (X-Y)/Y is lower than 3 and/or said fractional change (X-Y)/Y is lower than the corresponding mean fractional change as determined according to steps a) to d) in a cohort of control patients suffering from said inflammatory disease who are not responding to said therapy against inflammatory disease, then the signal output is such that it is predictive of a favourable response to therapy against inflammatory disease in the human subject suffering from said inflammatory disease was found.

The present invention also relates to a non-invasive method for predicting a response to a therapy against inflammatory disease for a human subject suffering from said inflammatory disease prior to application of said therapy against inflammatory disease using digital imaging techniques comprising the steps of:
a) collecting first digital imaging data indicative of neural activity from said human subject suffering from inflammatory disease under standardized control conditions and automatically determining, from said first digital imaging data, a value X of a significantly activated total brain area of said human subject suffering from inflammatory disease:
b) collecting second digital imaging data indicative of neural activity from said human subject suffering from inflammatory disease upon mechanical compression of a body part affected by said inflammatory disease and automatically determining, from said second digital imaging data, a value Y of a significantly activated total brain area of said human subject suffering from inflammatory disease;
c) calculating, from said determined values X and Y, a fractional change (X-Y)/Y;
d) automatically outputting, at an output unit, a signal representing said fractional change (X-Y)/Y;
   wherein if said fractional change (X-Y)/Y is lower than 3 and/or said fractional change (X-Y)/Y is lower than the corresponding mean fractional change as determined according to steps a) to d) in a cohort of control patients suffering from said inflammatory disease who are not responding to said therapy against inflammatory disease, then the signal output is such that it is predictive of a favourable response to therapy against inflammatory disease in the human subject suffering from said inflammatory disease.

The term "a significantly activated brain area" may be understood as an activated brain area determined according to the procedure described in example 1. In brief, after pre-processing (motion-correction using sinc interpolation Gaussian spatial (FWHM = 4 mm) and temporal (FWHM = 3 volumes) smoothing), Talairach transformation, resampling to 3x3x3 mm, and a multi-subject GLM analysis with separate predictors for each stimulus is performed. The SPM's obtained are corrected for multiple comparisons, FDR threshold at Z score-level of 3.3 (p < 0.001) and different groups of activated voxels (minimum of 4 voxels) are labeled as belonging to certain brain structures (Mai JK (2008) Elsevier Academic Press: Atlas of the human brain). The voxels which are significantly activated by the above criterion, are counted as the significantly activated volume per given brain region. The measurement can be performed by devices known in the art and is commercially available. One example of such a device is the 3 T scanner (Magnetom Trio, Siemens, Erlangen, Germany) using a standard 8-channel phased array head coil.

In summary, the invention shows that RA patients with high-level pain-related central nervous activity respond better to TNFi than those with low activation state. Importantly, clinical disease characteristics have been very similar among responders and non-responders highlighting previous failed attempts to predict response to TNFi by clinical and/or laboratory parameters. In fact, the data of the invention shape an entirely new concept, suggesting that the response to anti-inflammatory therapy, in particular cytokine blockade, depends on patients' disease perception rather than objective clinical features of disease.

The observation of the present invention that brain activity in the FMRI is linked to responsiveness to TNFi is very surprising, given that standard clinical measures fail to predict clinical responses to TNFi. It implies that these drugs are effective if the peripheral inflammatory process is adequately reflected in the central nervous system. This central nervous "mirror image" of disease, visualized by fMRI, likely reflects the perceived burden of RA, such as pain, fatigue and mood changes, rather than the activity of the inflammatory disease process itself. The observation that TNFi treatment works well in case of high central nervous representation of disease, suggests that TNFi essentially modulates disease perception. Importantly, it does so even before clinically measurable changes of objective disease symptoms can be recorded or structural effects on the joint such as reduction of synovitis can be found.

According to the present invention, treatment modalities, which influence disease perception, are favoured for reaching clinical responses, as compared to interventions, which show peripheral anti-inflammatory effects only. Changing perception of disease by drug therapy is desirable from the patients' perspective and one can speculate whether RA patients appreciate TNFi treatment at least partly because of central nervous effects, which shares similarities to central nervous system acting "uppers". In support of this concept, data of this invention shows that RA not only affects the activity of the pain pathway but also of other brain centers such as the prefrontal cortex, the insular region and the cingulate cortex, which are involved in mood changes including depression as well as memory. Importantly, central nervous system effects of cytokine blockers may differ among various diseases.

Strengths of the invention include the prospective, sequential and simultaneous analysis of brain activity, clinical disease activity and joint imaging in patients with RA, which allowed comparing the dymanics of CNS, clinical and structural responses, respectively.

In summary, the invention changes our understanding of the nature of the subjective burden of disease in RA patients. It provides explanations why TNFi treatment shows rapid and substantial effects on the patients overall disease state, which not only results from inflammation *per* se but also includes the effects of inflammation on the central nervous system. This invention allows the distinction of patients which respond well to TNFi- treatment from those which fail to experience clinical improvement of disease.

While the invention has been illustrated and described in detail in the drawings and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below.

The invention also covers all further features shown in the figures individually although they may not have been described in the afore or following description. Also, single alternatives of the embodiments described in the figures and the description and single alternatives of features thereof can be disclaimed from the subject matter of the other aspect of the invention.

Furthermore, in the claims the word "comprising" does not exclude other elements or steps, and the indefinite article "a" or "an" does not exclude a plurality. A single unit may fulfill the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

The present invention is also illustrated by the following figures.

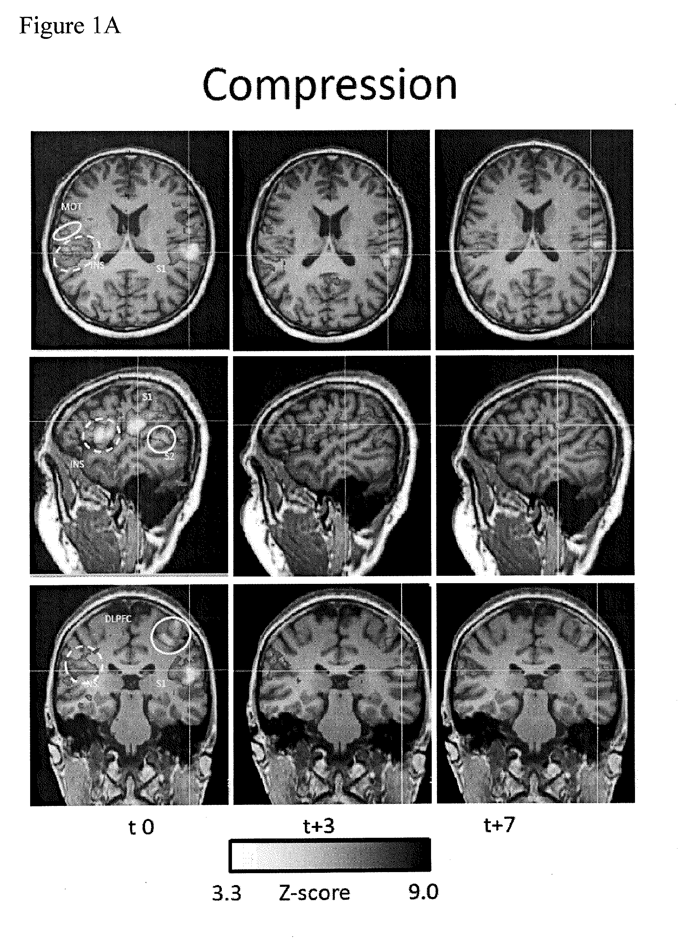

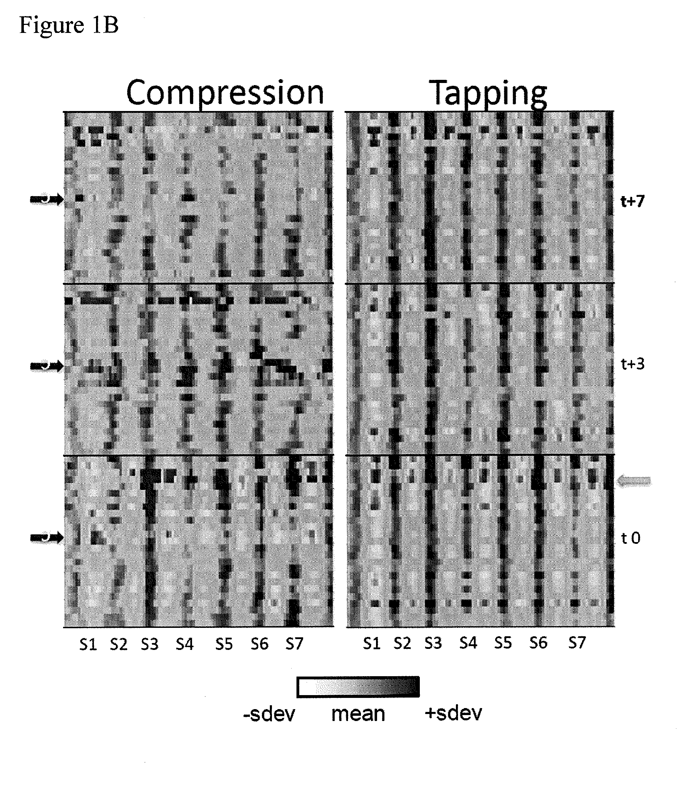

Figure 1. Rapid decrease of central nervous system activity upon tumor necrosis factor blockade
   (A.) Coronal (top), sagittal (middle) and axial (bottom) slices showing blood oxygen level dependent (BOLD) signals at brain structures activated by joint compression. Measurements were performed at baseline (t0) and 3 (t+3) and 7 days (t+7) after tumor necrosis factor blockade. S1: Primary somatosensory (white cross); S2: Secondary somatosensory; MOT: Motor; INS: Insular; DLPFC: Dorsolateral prefrontal cortex. (B.) BOLD signal time course during joint compression (left) and finger tapping (right) at baseline (t0) and 3 (t+3) and 7 days (t+7) after tumor necrosis factor blockade. X-axis: time, 7 stimulation (S) sequences (S1 to S7) are shown. Y-axis: each line represents one brain center (contra-and ipsilateral side next to each other); from bottom to top in the order: thalamus, primary and secondary somatosensory, parietal, posterior and anterior cingulate, medial and lateral prefrontal, anterior and posterior insular cortices, periaqueductal grey, cerebellum, motor cortex. Grey code: mean activity + and - 1 time standard deviation. Black arrows: somatosensory; purple arrow: medial prefrontal; grey arrow: motor cortex.
Figure 2. Disease activity of rheumatoid arthritis after exposure to tumor necrosis factor inhibitors
   Disease activity score (DAS) 28 in all patients with rheumatoid arthritis (A) and those responding (B) and not-responding (C) to tumor necrosis factor inhibitors. Asterisk indicates significant difference (p < 0.05).
Figure 3. Responders to tumor necrosis factor inhibition show higher central nervous system activity at baseline
   (A) Mean (± SEM) levels and (B.) changes of disease activity score (DAS) 28 at baseline and 28 days after tumor necrosis factor blockade in responders and non responders. (C) Absolute size and (D) relative change of activated brain areas as indicated by number of activated voxels (blood oxygen level dependent signal) in responders (solid line) and non-responders (dashed line). (E) 3-dimensional reconstructions of activated brain areas (Isosurfaces) at baseline and 3, 7 and 28 days after initiation of tumor necrosis factor blockade. Measurements were performed during joint compression and finger tapping (control procedure) in responders and non-responders.

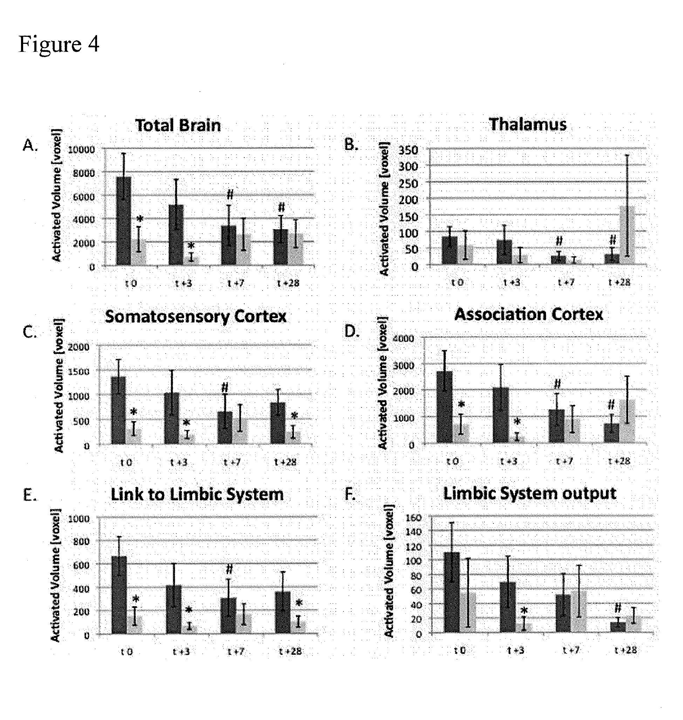

Figure 4. High baseline brain activity with consistent rapid decrease in patients responding to tumor necrosis factor inhibitors
   Activated brain volume (expressed in number of voxels) before (t0) and 3 days (t+3), 7 days (t+3) and 28 days (t+28) after exposure to tumor necrosis factor inhibitors in responders (dark) and no responders (bright). (A) total brain, (B) thalamus, (C) somatosensory cortex, (D) association cortex, (E) link to the limbic system and (F) limbic system output. Asterisks indicate significant (p < 0.05) differences between responders and non- responders, # indicate significant (p < 0.05) differences to the respective baseline level.

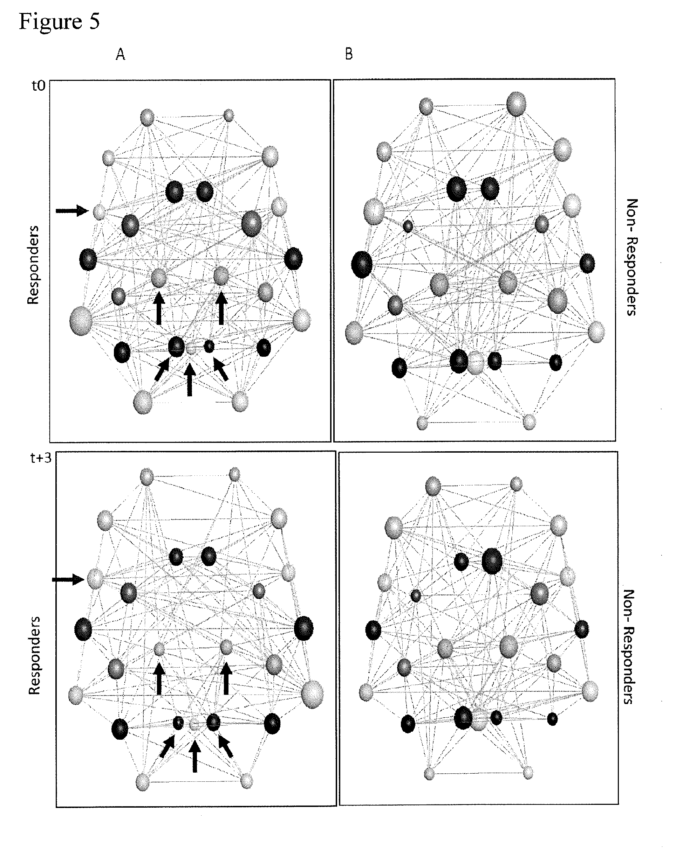

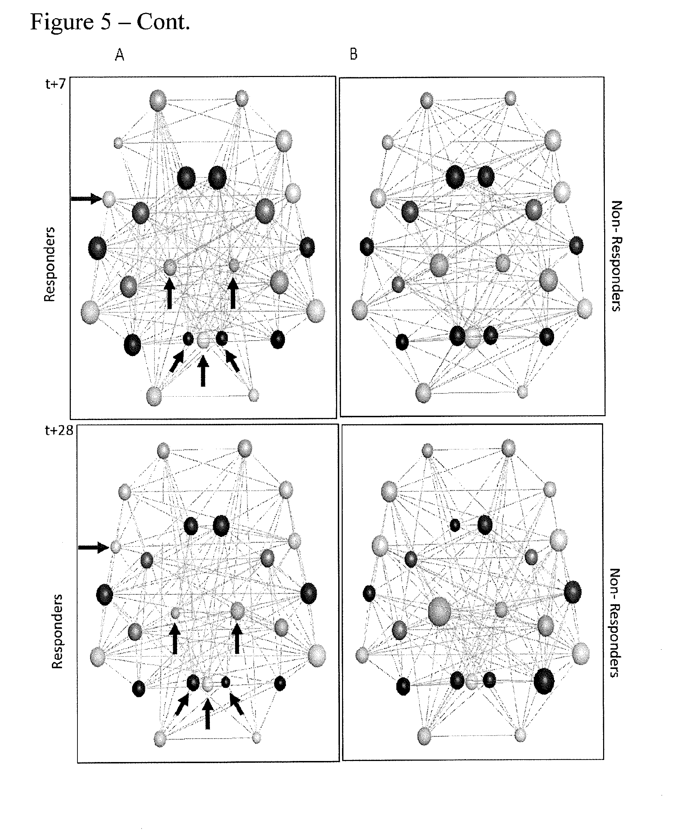

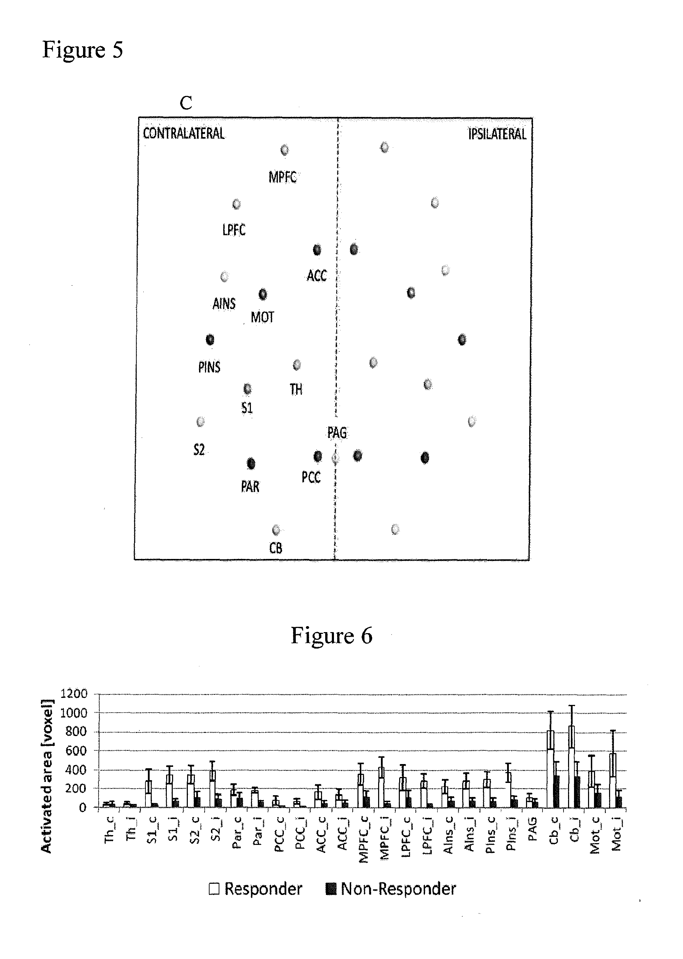

Figure 5. Graph theoretical analysis
   Connectivity networks related to pain (compression paradigm) of (A) responders and (B) non-responders before (t0), 3 days (t+3), 7 days (t+7) and 28 days (t+28) after application of certolizumab. Nodes represent brain structures and vertices the connectivity based on BOLD time course correlation between brain structures. Node size encodes the node's degree, isolated nodes (degree=0) are removed. Node positions are the anatomical centroids of the brain structures derived from a horizontal projection. Node colors represent different functional groups of brain structures. Vertex thickness encodes the connection strength (i.e. the correlation value). Arrows mark brain structures that show consistent differences in their connectivity between responders and non-responders (arrows in the center: thalamus, bottom arrows left and right: posterior cingulated cortex; top arrow: insular cortex; bottom arrow center: periaqueductal grey). (C) Brain structures analyzed for connectivity networks: (MPFC) medial prefrontal cortex, (LPFC) lateral prefrontal cortex, (AINS) anterior insular cortex, (PINS) posterior insular cortex, (MOT) motor cortex, (ACC) anterior cingulate cortex, (PCC) posterior cingulate cortex, (S1) primary somatosensory cortex, (S2) secondary somatosensory cortex, (TH) thalamus, (PAR) parietal cortex, (PAG) periaqueductal grey, (CB) cerebellum.
Figure 6. Activated brain areas
   The figure shows the brain structure specific activated area after compression of the metacarpophalangeal joint for non-responders (light bars) and responders (black bars). Plotted are the group average values with the standard error per structure contralateral (_c) and ipsilateral (i) to the side of stimulation: Th: thalamus, S1: primary somatosensory cortex, S2_ secondary somatosensory cortex, Par: parietal association cortex, PCC: posterior cingulate cortex, ACC: anterior cingulate cortex, MPFC: medial prefrontal cortex, LPFC: lateral prefrontal cortex, Alns: anterior insula, Plns: anterior insula, Cb: Cerebellum, Mot: motor cortex.

The present invention is additionally described by way of the following illustrative non-limiting examples that provide a better understanding of the present invention and of its many advantages. The following examples are included to demonstrate preferred embodiments of the invention. It should be appreciated by those of skill in the art that the techniques disclosed in the examples which follow represent techniques used in the present invention to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Examples

### Example 1: Materials and Methods

### Patients and Treatment

Ten patients with RA fulfilling the classification criteria for RA (14) were included into this study. All patients had failed on standard therapy with methotrexate and displayed moderate to highly active RA as defined by DAS28 score of more than 3.2. Patients received standard dose of the TNFi certolizumab pegol at a dose of 200mg by single subcutaneous injection. Sequential fMRI measurements as well as assessment of disease activity were undertaken in all patients at baseline and 3, 7 and 28 days after the onset of treatment. Peripheral MRI of the joints of the left hand was performed at baseline and after 28 days. The study was performed in accordance with the Declaration of Helsinki and approval from the ethics committee of the University Clinic of Erlangen as well as written informed consent from all individuals were obtained.

### Assessment of demographic and disease specific characteristics

Age, sex, autoantibody status (rheumatoid factor and anti-citrullinated protein antibodies) and disease duration were assessed in all individuals at baseline investigation. Clinical disease activity was assessed at baseline and after 3, 7 and 28 days by applying a validated score for measuring clinical disease activity (Disease Activity Score DAS 28), which is based on the assessment of the number of swollen and tender joints, the erythrocyte sedimentation rate and patients' rating of global disease condition on a visual analogue scale (6). Functional state of the patients was assessed by the Health Assessment Questionaire (15) at baseline and after 3, 7 and 28 days.

### MRI in humans

All MRI data were acquired on a 3 T scanner (Magnetom Trio, Siemens, Erlangen, Germany) using a standard 8-channel phased array head coil. For brain anatomy we used a T1 weighted MPRAGE-sequence (FOV = 256 mm, matrix size = 256 x 256, voxel size = 1.0 x 1.0 x 1.0 mm³, slices = 176, slice thickness = 1 mm, TR = 1900 ms, TE = 1.13 ms). For each subject, two functional experiments with different stimulation conditions (first, finger-tapping and second, compression of the left metacarpophalangeal joints) with 7 repetitions were performed. Overall105 whole-brain images were obtained with a gradient-echo, echo-planar scanning sequence (EPI; TR 3000 ms, TE 30 ms, flip angle 90°; FOV 192 mm x 192 mm acquisition matrix 128 x 128, 36 axial slices, slice thickness 3 mm, gap 0.75 mm), 7 volumes during stimulation and 8 volumes inter-stimulus-interval.

### Functional MRI analysis

Functional analysis was performed using Brain Voyager QX (Version 10.3) and our own software MagnAn as previously described in detail (4, 16). In summary, after pre-processing (motion-correction using sinc interpolation Gaussian spatial (FWHM = 4 mm) and temporal (FWHM = 3 volumes) smoothing), Talairach transformation, resampling to 3x3x3 mm, and a multi-subject GLM analysis with separate predictors for each stimulus was performed. The SPM's obtained were corrected for multiple comparisons, FDR threshold at Z score-level of 3.3 (p < 0.001) and different groups of activated voxels (minimum of 4 voxels) were labeled as belonging to certain brain structures (Mai JK (2008) Elsevier Academic Press: Atlas of the human brain). The voxels which were significantly activated by the above criterion, were counted as the activated volume per given brain region. The mean corresponding volume of brain structure specific activity was determined for tapping and compression, averaged over all activated brain structures and finally over all subjects within each group to provide a global group comparison.

### Connectivity analysis

Average time courses of the activated voxels of each brain structure were corrected for global signal fluctuations by linear regression. The residual time courses for all brain structures were cross-correlated individually for each patient and stimulus. The resulting cross-correlation matrices were averaged over all responders and non-responders for each stimulus separately. Based on these mean cross-correlation matrices, network graphs were obtained with correlation coefficients coding the thickness of the edges and brain structures as the nodes. The individual node degree was calculated and used as radius for plotting each node. Isolated nodes (degree = 0) were removed. The horizontal projections of the 3D centroids of the brain structures defined the node positions. The nodes were coded based on their functional membership.

### Statistical analysis

Data are presented as mean ± SEM. Group means values were compared by the Mann Whitney U test. A P value of less than 0.05 was considered as statistically significant.

### Experimental set-up

Patients received standard subcutaneous dosing of TNFi and sequential FMRI measurements were undertaken at baseline and 3, 7 and 28 days after the onset of treatment. Clinical disease activity was assessed at the same time points by applying a validated score for measuring clinical disease activity (Disease Activity Score DAS 28) in RA which is based on the assessment of the number of swollen and tender joints, the erythrocyte sedimentation rate and patients' rating of global disease condition by visual analogue scale (5). Furthermore, inflammatory tissue was directly assessed by anatomical MRI of the dominantly affected hand at baseline and after 28 days (18).

### Example 2 Rapid decrease of disease-related fMRI brain activity in RA patients by TNFi

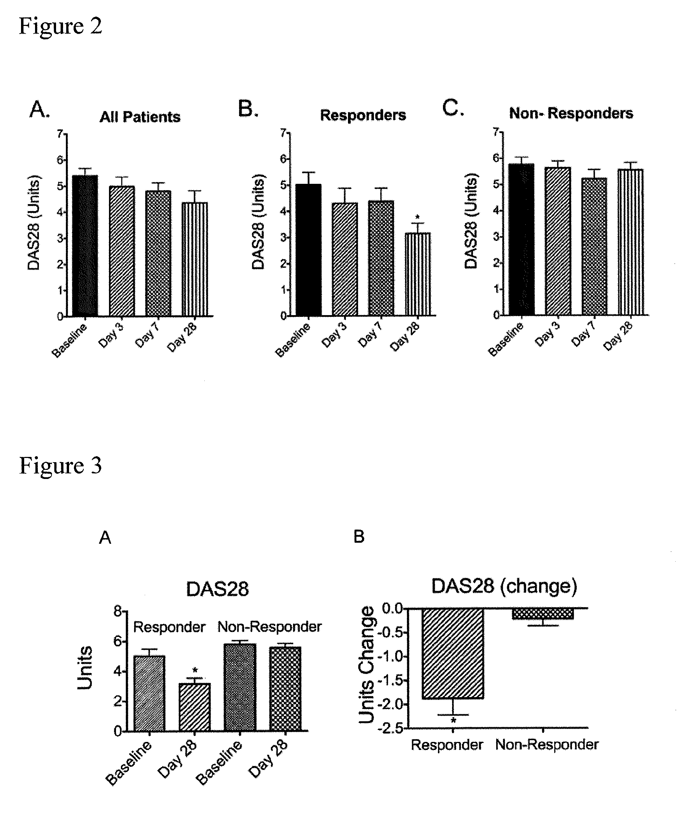

Assessment of BOLD activity at baseline revealed high signal intensity at areas of the somatosensory cortex, the insular and dorsolateral prefrontal cortex (Figure 1A). This activation was already reduced as early as 3 days after exposure to TNFi and further decreased 7 days after the initiation of treatment. Detailed mapping of neuronal activity, as shown in Figure 1B, showed complete abrogation of hot spots of BOLD activity in the somatosensory cortex, the prefrontal cortex and the limbic system as early as 3 days after exposure to TNFi and persistent low activity in these centers 7 days after initiation of the treatment (Figure 1B, Compression). In contrast, neuronal activity elicited by finger tapping, used as control procedure, was only marginally affected by TNFi and showed the expected dominant activation of the motor cortical areas (Figure 1B, Tapping). Importantly, the observed rapid decrease in neuronal activity after exposure to TNFi preceded both clinical and structural responses to the drug. Thus, clinical disease activity did not change 3 and 7 after exposure to TNFi (Figure 2), but only as early as 28 days after the start of the treatment. Routine anatomical MRI examination of the joints even showed unchanged inflammatory infiltrates 28 days after exposure to TNFi (see Table 1) clearly indicating that central nervous system activity in RA decreases before any known peripheral anti-inflammatory effects become measurable.

**Table 1 Anatomical magnetic resonance imaging of the right hand**

| **Parameter** | **Baseline** | **Day 28** | **p-value** |
|---|---|---|---|
| Bone Marrow Inflammation (Units) | 1.60 ± 0.87 | 0.80 ± 0.20 | 0.69 |
| Synovial Inflammation (Units) | 5.20 ± 1.46 | 5.00 ± 0.54 | 0.74 |
| | | | |

### Example 3: High disease-related fMRI brain activity in responders to TNFi as compared to non-responders

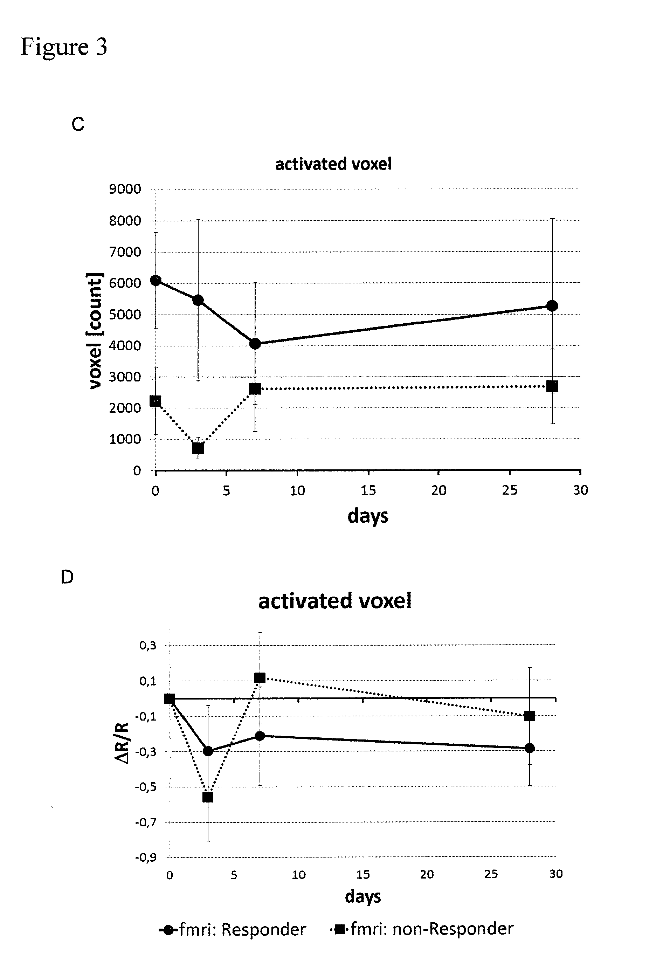

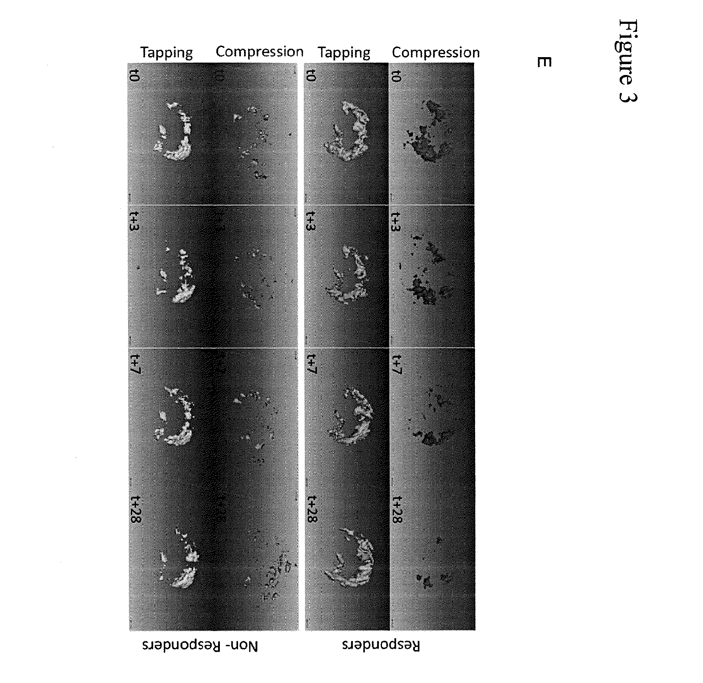

Among the 10 patients included in this study, five developed significant clinical improvement of disease activity after 28 days (mean ± SEM change in DAS 28 score -1.8± 0.3), whereas the other 5 patients did not improve at all (mean ± SEM change in DAS 28 score: -0.2± 0.1). Importantly baseline disease activity (responders 5.1± 0.5; non-responders 5.7± 0.3; Figure 3A and B) was not different among the two groups and also other demographic and disease specific parameters, like age, gender distribution, autoantibody positivity, physical function as well as disease duration, did not show any significant differences at baseline (Table 2). Responders, however, showed significantly higher activated brain volume than non-responders in the baseline fMRI examination and during all follow-up visits (Figure 3C). Furthermore, BOLD signals in the brain decreased consistently in the responder group with signals lower than baseline after 3, 7 and 28 days (Figure 3D and E). In contrast, non-responders did only show a spurious suppression of neuronal activity after 3 days which regressed to baseline levels after 7 and 28 days. Of note, even such an easy accessible parameter like the overall activated voxels allowed the differentiation in responders and non-responders (Figure 4A). Moreover, also the activity of individual brain centers, particularly the somatosensory cortex, the associative cortex and the limbic system allowed differentiation between responders and non responders, with responders showing higher baseline activity decreasing consistently after exposure to TNFi (Figure 4B-F).

**Table 2 Demographic and disease-specific characteristics at baseline**

| **Parameter** | **Responders** | **Non-Responders** | **p-value** |
|---|---|---|---|
| Age (years) | 50.4 ± 4.4 | 61.2 ± 5.9 | 0.31 |
| Disease Duration (years) | 31.2 ± 8.1 | 27.2 ± 5.3 | 0.17 |
| Tender Joints (N) | 13.6 ± 3.5 | 15.2 ± 2.2 | 0.69 |
| Swollen Joints (N) | 10.4 ± 2.1 | 9.8 ± 1.3 | 0.75 |
| Visual Analogue Scale Pain (mm) | 49.0 ± 7.8 | 66.0 ± 4.0 | 0.10 |
| Erythrocyte Sedimentation Rate (mm) | 27.0 ± 5.7 | 37.2 ± 16.7 | 0.91 |
| Disease Activity Score 28 (Units) | 5.0 ± 0.5 | 5.7 ± 0.3 | 0.40 |
| Health Assessment Questionaire (Units) | 1.02 ± 0.43 | 1.10 ± 0.22 | 0.39 |

### Example 4: Differences in the connectivity of brain centers among TNFi -responders and non-responders

Furthermore analysis of the connectivity of the different brain centers by graph theoretical approaches revealed additional differences between responders and non-responders: Connectivity of thalamic centers (Figure 5A and B, black arrows in the center) which are primarily involved in pain perception, and of the periaquaeductal grey (Figure 5A and B, black arrow bottom center), which elicits efferent anti-nociceptive signals were consistently higher in non-responders than in responders both at baseline and during the entire follow up period. Preferential involvement of efferent anti-nociceptive signals in the non-responders could explain their overall lower cortical neuronal activity and the failure of TNFi to affect this process. Additional differences in the connectivity of brain centers between TNFi responders and non-responders affected were observed in brain structures involved in pain rating and in the regulation of mood, such as the insular region, (Figure 5A and B; black arrow top) and those involved in memory and pain such as the posterior cingulate cortex (Figure 5A and B, black arrows bottom right and left). These findings are particularly relevant in the light of recent findings showing changes in the anatomical distribution of the grey matter in the brain of patients with RA, particularly involve centers important for pain processing and behavior.

### Example 5: Determination of the fractional change (X-Y)/Y in a cohort of non-responders

The present invention allows determining the fractional change (X-Y)/Y according to the steps (a) to (d) of any of the methods provided by the present invention in a cohort of control patients suffering from said inflammatory disease who are not responding to said therapy against inflammatory disease. For example, steps (a) to (d) of any method of the present invention are performed with a group of 10-10000 patients suffering from the respective inflammatory disease under consideration of the respective treatment against said inflammatory disease. In this example, all patients suffer from the same inflammatory disease. After performing steps (a) to (d), the patients would be treated with the therapy against the inflammatory disease under consideration. For this example, the treatment against the inflammatory disease is the same for all patients. After an adequate period of time, known by the person skilled in the art to assume that a (desirable) response to the treatment can be expected, the patients can be listed in two groups:
(a) therapy responders
(b) therapy non-responders
by an assessment of the therapy response for each patient according to standard criteria known to the person skilled in the art which are independent of the present invention. Then, the mean value of the fractional changes (X-Y)/Y of the patients of group (b) can be determined and will provide a reference value for methods of the present invention.

### Example 6: Determination of the fractional change (X-Y)/Y in a cohort of RA patients being non-responders to TNFi treatment

The present invention allows determining the fractional change (X-Y)/Y according to the steps (a) to (d) of respective methods provided by the present invention in a cohort of control patients suffering from active RA who are not responding to TNFi treatment. For example, steps (a) to (d) of any method of the present invention can be performed for a group of 10-10000 patients suffering from active RA. After performing steps (a) to (d), the patients can be treated with TNFi as known to the person skilled in the art. After an adequate period of time, known by the person skilled in the art, e.g. after 28 days as illustrated for example by figure 2 and outlined by example 2, the patients could be listed in to groups:
(a) TNFi therapy responders
(b) TNFi therapy non-responders
   by an assessment according to standard criteria known to the person skilled in the art, e.g. by evaluation of the DAS28 score. Then, the mean value of the fractional changes (X-Y)/Y of the patients of group (b) can be determined and would provide a reference value for methods of the present invention.

### Example 7: Application example of the present invention

From patient GK after appropriate preprocessing of the functional imaging data as outlined above and after FDR thresholding the statistical parameter maps at 3.3 a value of 26963 significantly activated voxels (cf. Fig. 3E, Tapping, t0, responders) within the brain for the finger tapping task, which is the value X, was obtained. The second experiment with the mild painful stimulation resulted in an activity of 9221 significantly activated voxels (cf. Fig. 3E, Compression, t0, responders) within the brain after appropriate preprocessing of the functional imaging data as outlined above and after FDR thresholding the statistical parameter maps at 3.3. Following the formula given in the method step d of the methods of the present invention an output value of 1.92 fractional change was obtained, which assigns patient GK to the responder group.

For a second patient (BJ) a value of 22972 significantly activated voxels within the brain (cf. Fig. 3E, Tapping, t0, non-responders) for value X, the finger tapping task, was automatically obtained after appropriate preprocessing of the functional imaging data as outlined above and after FDR thresholdind the statistical parameter maps at 3.3 as well as a value 4246 voxels within the brain (cf. Fig. 3E, Compression, t0, non-responders) for the mild painful task, which is value Y. Following the formula given in claim the method step d of the methods of the present invention a value of 4.41 fractional change was obtained as the output value, which assigns the second patient to the group of non-responders.

## Claims

1. A non-invasive method for predicting a response to a therapy against inflammatory disease for a human subject suffering from said inflammatory disease prior to application of said therapy against inflammatory disease using digital imaging techniques comprising the steps of:
a) collecting first digital imaging data indicative of neural activity from said human subject suffering from inflammatory disease under standardized control conditions and automatically determining, from said first digital imaging data, a value X of an activated total brain area of said human subject suffering from inflammatory disease:
b) collecting second digital imaging data indicative of neural activity from said human subject suffering from inflammatory disease upon mechanical compression of a body part affected by said inflammatory disease and automatically determining, from said second digital imaging data, a value Y of an activated total brain area of said human subject suffering from inflammatory disease;
c) calculating, from said determined values X and Y, a fractional change (X-Y)/Y;
d) automatically outputting, at an output unit, a signal representing said fractional change (X-Y)/Y;
wherein if said fractional change (X-Y)/Y is lower than 3 and/or said fractional change (X-Y)/Y is lower than the corresponding mean fractional change as determined according to steps a) to d) in a cohort of control patients suffering from said inflammatory disease who are not responding to said therapy against inflammatory disease, then the signal output is such that it is predictive of a favourable response to therapy against inflammatory disease in the human subject suffering from said inflammatory disease.

2. A non-invasive method for predicting a response to a therapy against inflammatory disease for a human subject suffering from said inflammatory disease prior to application of said therapy against inflammatory disease using digital imaging techniques comprising the steps of:
a) collecting first digital imaging data indicative of neural activity from said human subject suffering from inflammatory disease under standardized control conditions and automatically determining, from said first digital imaging data, a value X of an activated total brain area of said human subject suffering from inflammatory disease:
b) collecting second digital imaging data indicative of neural activity from said human subject suffering from inflammatory disease upon mechanical compression of a body part affected by said inflammatory disease and automatically determining, from said second digital imaging data, a value Y of an activated total brain area of said human subject suffering from inflammatory disease;
c) calculating, from said determined values X and Y, a fractional change (X-Y)/Y;
d) automatically outputting, at an output unit, a signal representing said fractional change (X-Y)/Y;
wherein if said fractional change (X-Y)/Y is lower than 3, then the signal output is such that it is predictive of a favourable response to therapy against inflammatory disease in the human subject suffering from said inflammatory disease.

3. A non-invasive method for predicting a response to a therapy against inflammatory disease for a human subject suffering from said inflammatory disease prior to application of said therapy against inflammatory disease using digital imaging techniques comprising the steps of:
a) collecting first digital imaging data indicative of neural activity from said human subject suffering from inflammatory disease under standardized control conditions and automatically determining, from said first digital imaging data, a value X of an activated total brain area of said human subject suffering from inflammatory disease:
b) collecting second digital imaging data indicative of neural activity from said human subject suffering from inflammatory disease upon mechanical compression of a body part affected by said inflammatory disease and automatically determining, from said second digital imaging data, a value Y of an activated total brain area of said human subject suffering from inflammatory disease;
c) calculating, from said determined values X and Y, a fractional change (X-Y)/Y;
d) automatically outputting, at an output unit, a signal representing said fractional change (X-Y)/Y;
wherein, if said fractional change (X-Y)/Y is lower than the corresponding mean fractional change as determined according to steps a) to d) in a cohort of control patients suffering from said inflammatory disease who are not responding to said therapy against inflammatory disease, then such is predictive of a favourable response to said therapy against inflammatory disease in said human subject suffering from said inflammatory disease.

4. The non-invasive method of claim 1 or 3 wherein said inflammatory disease is rheumatoid arthritis, psoriatic arthritis, spondyloarthritis or inflammatory bowel disease.

5. The non-invasive method of claims 1 to 4 wherein said imaging technique is fMRI.

6. The non-invasive method of claim 5, wherein said imaging data indicative of neural activity is blood-oxygen level dependent (BOLD), arterial spin labelling (ASL), cerebral blood flow (CBF), or cerebral blood volume (CBV) MRI data.

7. The non-invasive method of claims 1 to 4 wherein the imaging technique is PET.

8. The non-invasive method of claim 7 wherein the imaging data indicative of neural activity is ¹⁸FDG-PET or ¹⁵O-PET data.

9. The non-invasive method of any of claims 1 to 8 wherein said therapy against inflammatory disease is administration of an antagonist of TNFalpha, IL-6R, IL-6 or IL-17.

10. The non-invasive method of any of claims 1 to 9 wherein said therapy against inflammatory disease is administration of a pharmaceutical composition comprising an agent selected from the group comprising Adalimumab, Certolizumab pegol, Etanercept, Golimumab, Infliximab, Tocilizumab, Anakinra, Rituximab, Abatacept and Secukinumab.

11. The non-invasive method of any of claims 1 to 10, wherein said inflammatory disease is rheumatoid arthritis or psoriatic arthritis and wherein said affected body part is a metacarpophalangeal joint.

12. The non-invasive method of any of claims 1 to 10, wherein said inflammatory disease is inflammatory bowel disease, wherein said affected body part is the abdomen.

13. The non-invasive method of any of claims 1 to 12, wherein said standardized control conditions comprise the process of finger-tapping of said human subject.

14. A pharmaceutical composition comprising an antibody targeting TNFalpha, IL-6R, IL-6 or IL-17 as active ingredient for use in treating a human subject suffering from inflammatory disease wherein for said human subject a favourable response to therapy has been predicted according to the method of any of claims 1 to 13.

15. A pharmaceutical composition comprising an agent selected from the group comprising Adalimumab, Certolizumab pegol, Etanercept, Golimumab, Infliximab, Tocilizumab, Anakinra, Rituximab, Abatacept and Secukinumab for use in treating a human subject suffering from inflammatory disease wherein for said human subject a favourable response to therapy has been predicted according to the method of any of claims 1 to 14 wherein said inflammatory disease is rheumatoid arthritis.
